# EUROPEAN PATENT APPLICATION

(11) **EP 1 795 531 A1**
(43) Date of publication of application: **13.06.2007**
(21) Application number: 07105370.6
(22) Date of filing: 28.09.2001
(51) Int. Cl.: C07D 417/12, A61K 31/4439, A61P 3/10

(54) **A thiazolidinedione derivative and its use as antidiabetic**

(30) Priority: 29.09.2000 GB 0023970
(62) Divisional of application: 01972254.5
(71) Applicant: SMITHKLINE BEECHAM PLC, Brentford, Middlesex TW8 9GS (GB)
(72) Inventor: Millan, Michael, Tonbridge, Kent TN11 9AN (GB)
(74) Representative: Stevenson, Dian Elizabeth

(57) **Abstract**

A compound 5-[4-[2-(N-methyl-N-(2-pyridyl)amino)ethoxy]benzyl]thiazolidine-2,4-dione potassium salt or a pharmaceutically acceptable solvate thereof, a pharmaceutical composition containing such a compound and the use of such a compound in medicine, particularly in the treatment of diabetes mellitus.

## Description

This invention relates to a novel pharmaceutical, to a process for the preparation of the pharmaceutical and to the use of the pharmaceutical in medicine.

European Patent Application, Publication Number 0,306,228 relates to certain thiazolidinedione derivatives disclosed as having hypoglycaemic and hypolipidaemic activity. The compound of example 30 of EP 0,306,228 is 5-[4-[2-(N-methyl-N-(2-pyridyl)amino)ethoxy]benzyl]thiazolidine-2,4-dione (hereinafter also referred to as "Compound (I)").

International Patent Application, Publication Number WO94/05659 discloses certain salts of the compounds of EP 0,306,228. The preferred salt of WO94/05659 is the maleic acid salt.

It has now been discovered that Compound (I) forms a novel potassium salt (hereinafter also referred to as the "Potassium Salt") that is particularly stable and hence is suitable for bulk preparation and handling. The Potassium Salt also has a high melting point and shows particularly good aqueous solubility. The potassium Salt is therefore surprisingly amenable to large scale pharmaceutical processing and especially to large scale milling. The novel salt can be prepared by an efficient, economic and reproducible process particularly suited to large-scale preparation.

The novel Potassium Salt also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione, potassium salt or a solvate thereof.

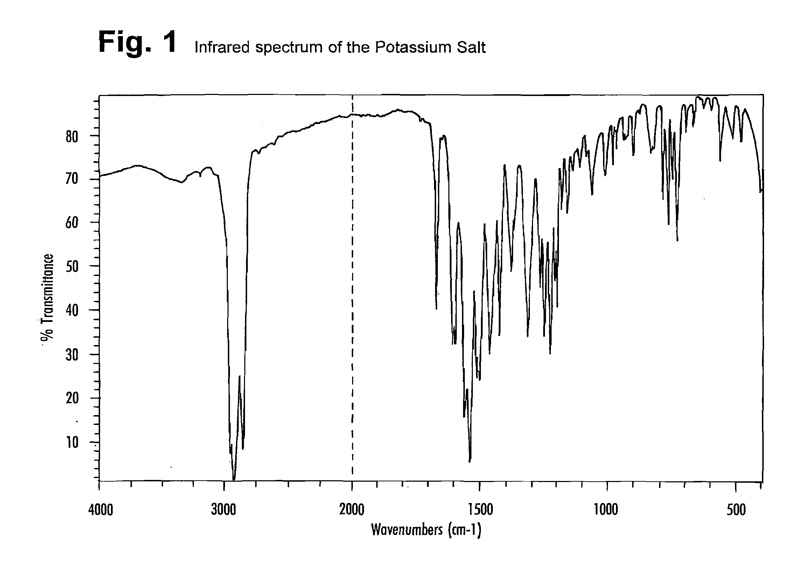

In one favoured aspect, the Potassium Salt provides an infrared spectrum substantially in accordance with Figure 1.

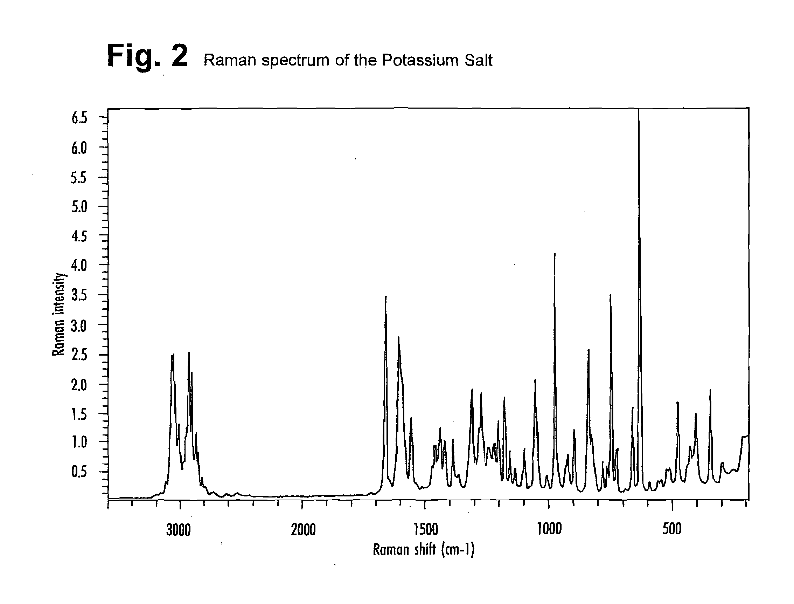

In one favoured aspect, the Potassium Salt provides a Raman spectrum substantially in accordance with Figure 2.

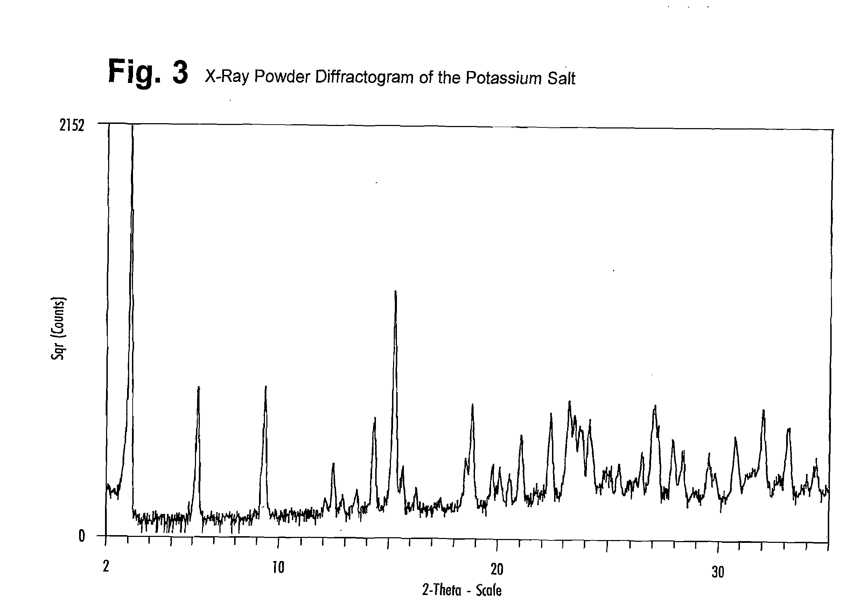

In one favoured aspect, the Potassium Salt provides an X-Ray powder diffraction pattern (XRPD) substantially in accordance with Table 1 or Figure 3.

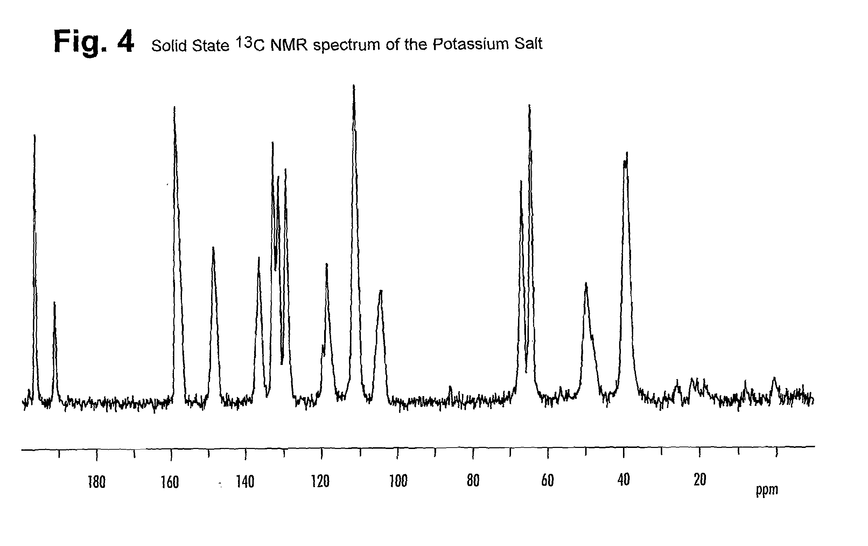

In one favoured aspect, the Potassium Salt provides a Solid State ¹³C NMR spectrum substantially in accordance with Figure 4.

In a preferred aspect, the invention provides 5-[4-[2-(N-methyl-N-(2-pyridyl)amino)ethoxy]benzyl]thiazolidine-2,4-dione, potassium salt, or a solvate thereof characterised in that it provides:
(i) an infrared spectrum substantially in accordance with Figure 1; and
(ii) a Raman spectrum substantially in accordance with Figure 2; and
(iii) an X-Ray powder diffraction pattern (XRPD) substantially in accordance with Table 1 or Figure 3; and
(iv) a Solid State ¹³C NMR spectrum substantially in accordance with Figure 4.

The present invention encompasses the Potassium Salt or solvate thereof isolated in pure form or when admixed with other materials. Thus in one aspect there is provided the Potassium Salt or solvate thereof in isolated form.

In a further aspect there is provided the Potassium Salt or solvate thereof in a purified form.

In yet a further aspect there is provided the Potassium Salt or solvate thereof in crystalline form.

Also, the invention provides the Potassium Salt or solvate thereof in a solid pharmaceutically acceptable form, such as a solid dosage form, especially when adapted for oral administration.

Moreover, the invention also provides the Potassium Salt or solvate thereof in a pharmaceutically acceptable form, especially in bulk form, such form being particularly capable of being milled. The invention also provides the Potassium Salt in a milled form.

A suitable solvate is a hydrate.

The invention also provides a process for preparing the Potassium Salt or a solvate thereof, characterised in that 5-[4-[2-(N-methyl-N-(2-pyridyl)amino)ethoxy]benzyl]thiazolidine-2,4-dione (Compound (I)) or a salt thereof, preferably dispersed or dissolved in a suitable solvent, is reacted with a source of potassium ion and thereafter, if required, a solvate of the resulting Potassium Salt is prepared; and the Potassium Salt or a solvate thereof is recovered.

A suitable reaction solvent is an alkanol, for example propan-2-ol, or a hydrocarbon, such as toluene, a ketone, such as acetone, an ester, such as ethyl acetate, an ether such as tetrahydrofuran, a nitrile such as acetonitrile, or a halogenated hydrocarbon such as dichloromethane, or water; or a mixture thereof.

Conveniently, the source of potassium ion is potassium hydroxide. The potassium hydroxide is preferably added as a solid or in solution, for example in water or a lower alcohol such as methanol, ethanol, or propan-2-ol, or a mixture of solvents. An alternative source of potassium ion is a potassium alkoxide salt for example potassium tertiary-butoxide.

The concentration of Compound (I) is preferably in the range 2 to 25% weight/volume, more preferably in the range 5 to 20%. The concentration of potassium hydroxide solutions are preferably in the range of 2 to 110% weight/volume.

The reaction is usually carried out at ambient temperature or at an elevated temperature, for example at the reflux temperature of the solvent, although any convenient temperature that provides the required product may be employed.

Solvates, such as hydrates, of the Potassium Salt are prepared according to conventional procedures.

Recovery of the required compound generally comprises crystallisation from an appropriate solvent, conveniently the reaction solvent, usually assisted by cooling. For example, the Potassium Salt may be crystallised from an alcohol such propan-2-ol, a ketone such as acetone, an ester such as ethyl acetate, an ether such as tetrahydrofuran or water or a mixture thereof. An improved yield of the salt can be obtained by evaporation of some or all of the solvent or by crystallisation at elevated temperature followed by controlled cooling, optionally in stages. Careful control of precipitation temperature and seeding may be used to improve the reproducibility of the product form.

Crystallisation can also be initiated by seeding with crystals of the Potassium Salt or a solvate thereof but this is not essential.

Compound (I) is prepared according to known procedures, such as those disclosed in EP 0,306,228 and WO94/05659. The disclosures of EP 0,306,228 and WO94/05659 are incorporated herein by reference.

Potassium hydroxide and potassium tertiary-butoxide are commercially available compounds.

When used herein the term "Tₒₙₛₑₜ" is generally determined by Differential Scanning Calorimetry and has a meaning generally understood in the art, as for example expressed in Pharmaceutical Thermal Analysis, Techniques and Applications", Ford and Timmins, 1989 as "The temperature corresponding to the intersection of the pretransition baseline with the extrapolated leading edge of the transition".

When used herein the term prophylaxis of conditions associated with diabetes mellitus includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides the Potassium Salt or a solvate thereof for use as an active therapeutic substance.

More particularly, the present invention provides the Potassium Salt or a solvate thereof for use in the treatment and/or prophylaxis of diabetes mellitus, conditions associated with diabetes mellitus and certain complications thereof.

The Potassium Salt or a solvate thereof may be administered per se or, preferably, as a pharmaceutical composition also comprising a pharmaceutically acceptable carrier. Suitable methods for formulating the Potassium Salt or a solvate thereof are generally those disclosed for Compound (I) in the publications mentioned herein..

Accordingly, the present invention also provides a pharmaceutical composition comprising the Potassium Salt or a solvate thereof and a pharmaceutically acceptable carrier therefor

The Potassium Salt or a solvate thereof is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of Potassium Salt or a solvate thereof to a human or non-human mammal in need thereof.

Conveniently, the active ingredient may be administered as a pharmaceutical composition hereinbefore defined, and this forms a particular aspect of the present invention.

In a further aspect the present invention provides the use of Potassium Salt or a solvate thereof for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof the Potassium Salt or a solvate thereof may be taken in amounts so as to provide Compound (I) in suitable doses, such as those disclosed in EP 0,306,228, WO94/05659 or WO98/55122.

The unit dose compositions of the invention comprise the Potassium Salt or a pharmaceutically acceptable solvate thereof in an amount providing up to 12mg, including 1-12mg such as 2-12mg of Compound (I), especially 2-4mg, 4-8mg or 8-12mg of Compound (I), for example 1,2, 3, 4, 5,6, 7, 8,9,10,11 or 12mg of Compound (I). Thus in particular there is provided a pharmaceutical composition comprising the Potassium Salt or a solvate thereof and a pharmaceutically acceptable carrier therefor, wherein the Potassium Salt or a pharmaceutically acceptable solvate thereof is present in an amount providing 1, 2, 4, 8, 4 to 8 or 8 to 12mg of Compound (I); such as 1mg of Compound (I); such as 2mg of Compound (I); such as 4mg of Compound (I); such as 8mg of Compound (I); such as 12mg of Compound (I).

The invention also provides a pharmaceutical composition comprising the Potassium Salt or a pharmaceutically acceptable solvate thereof in combination with one or more other anti-diabetic agents and optionally a pharmaceutically acceptable carrier therefor.

The invention also provides a method for the treatment and/or prophylaxis of diabetes mellitus, conditions associated with diabetes mellitus and certain complications thereof, in a human or non-human mammal which comprises administering an effective, non-toxic, amount of the Potassium Salt or a pharmaceutically acceptable solvate thereof in combination with one or more other anti-diabetic agents.

In a further aspect the present invention provides the use of the Potassium Salt or a pharmaceutically acceptable solvate thereof in combination with one or more other anti-diabetic agents, for the manufacture of a medicament for the treatment and/or prophylaxis of diabetes mellitus, conditions associated with diabetes mellitus and certain complications thereof.

In the above mentioned treatments the administration of the Potassium Salt or a pharmaceutically acceptable solvate thereof and the other anti-diabetic agent or agents includes co-administration or sequential administration of the active agents.

Suitably in the above mentioned compositions, including unit doses; or treatments the Potassium Salt or a pharmaceutically acceptable solvate thereof is present in an amount providing up to 12mg, including 1-12mg, such as 2-12mg of Compound (I), especially 2-4mg, 4-8mg or 8-12mg of Compound (I), for example 1, 2, 3, 4, 5, 6, 7, 8, 9, 10, 11, or 12mg of Compound (I) or 4 to 8 or 8 to 12 mg of Compound (I). Thus for example in the above mentioned compositions, including unit doses, or treatments the Potassium Salt or a pharmaceutically acceptable solvate thereof is present in an amount providing 1mg of Compound (I); the Potassium Salt or a pharmaceutically acceptable solvate thereof is present in an amount providing 2mg of Compound (I); the Potassium Salt or a pharmaceutically acceptable solvate thereof is present in an amount providing 3mg of Compound (I); the Potassium Salt or a pharmaceutically acceptable solvate thereof is present in an amount providing 4mg of Compound (I); or the Potassium Salt or a pharmaceutically acceptable solvate thereof is present in an amount providing 8mg of Compound (I).

The other antidiabetic agents are suitably selected from biguanides, sulphonylureas and alpha glucosidase inhibitors. The other antidiabetic agent is suitably a biguanide. The other antidiabetic agent is suitably a sulphonylureas. The other antidiabetic agent is suitably a alpha glucosidase inhibitor. Suitable antidiabetic agents are those disclosed in WO98/57649, WO98/57634, WO98/57635, WO98/57636, WO99/03477, WO99/03476.

The contents of the above mentioned publications are incorporated herein by reference.

No adverse toxicological effects are indicated in the above mentioned treatments for the compounds of the invention.

The following examples illustrate the invention but do not limit it in any way.

### EXAMPLES

### Example 1 5-[4-[2-(N Methyl-N-(2-pyridyl)amino)ethoxy]benzyl]thiazolidine-2,4-dione, potassium salt

A solution of potassium hydroxide (0.56 g) in water (5 ml) was added to a stirred solution of 5-[4-[2-(*N*-methyl-*N*-(2-pyridyl)amino)ethoxy]benzyl]thiazolidine-2,4-dione (3.0 g) in tetrahydrofuran (30 ml) at 50°C. The solution was cooled with stirring to 21°C over approximately 1 hour, before the solvent was evaporated under reduced pressure to afford 5-[4-[2-(*N*-methyl-*N*-(2-pyridyl)amino)ethoxy]benzyl]thiazolidine-2,4-dione, potassium salt (2.90 g) as a crystalline solid.

### Example 2 5-[4-[2-(N-Methyl-N-(2-pyridyl)amino)ethoxy]beuzyl]thiazolidine-2,4-dione, potassium salt

A stirred suspension of 5-[4-[2-(N-methyl-N-(2-pyridyl)amino)ethoxy]benzyl] thiazolidine-2,4-dione (3.0 g) in acetone (30 ml) was heated to reflux before a solution of potassium hydroxide (0.56 g) in water (5 ml) was added. After 5 minutes a clear solution was formed and the temperature of the stirred solution was lowered to 21°C over approximately 1 hour. The solvent was evaporated under reduced pressure to give the 5-[4-[2-(*N*-methyl-*N*-(2-pyridyl)amino)ethoxy]benzyl]thiazolidine-2,4-dione, potassium salt (3.25 g) as a crystalline solid.

### Example 3 5-[4-[2-(N-Methyl-N-(2-pyridyl)amino)ethoxy]benzyl]thiazolidine-2,4-dione, potassium salt

A solution of potassium hydroxide (0.56 g) in water (1 ml) was added to a stirred suspension of 5-[4-[2-(N-methyl-N (2-pyridyl)amino)ethoxy]benzyl]thiazolidine-2,4-dione (3.D g) in propan-2-ol (30 ml) at reflux. Within 5 minutes the solution became clear before a precipitate began to form. The stirred mixture was cooled to 21°C over approximately 90 minutes. The solid precipitate was collected by filtration, washed with propan-2-ol (10 ml) and dried under vacuum for 16 hours to afford 5-[4-[2-(*N* methyl-*N* (2-pyridyl)amino)ethoxy]benzyl]thiazolidine-2,4-dione, potassium salt (3.14 g) as a white crystalline solid.
Found (%): C: 54.44, H: 4.53, N: 10.45; Expect: C: 54.52, H: 4.83, N: 10.60.

The potassium ion level was determined as 9.9% by wt (expect: 9.9%) by ion chromatography.
Water content (Karl-Fisher): 0.2 % by wt.

### Example 4 S-[4-[2-(N Methyl N (2-pyridyl)amino)ethoxy]benzyl]thiazolidine-2,4-dione, potassium salt

Potassium *t*-butoxide (1.41 g) was added to a stirred suspension of 5-[4-[2-(*N*-methyl-*N-*(2-pyridyl)amino)ethoxy]benzyl]thiazolidine-2,4-dione (3.0 g) in ethyl acetate (30 ml) at reflux. The stirred mixture was maintained at reflux for 15 minutes and then cooled to 21 °C over approximately 1 hour. The solid was collected by filtration, washed with ethyl acetate (10 ml) and dried under vacuum at 50°C for 72 hours to yield the 5-[4-[2-(N-methyl-*N*-(2-pyridyl)amino)ethoxy]benzyl]thiazolidine-2,4-dione, potassium salt (3.30 g) as a white crystalline solid.

### Example 5 5-[4-[2-(N-Methyl-N-(2-pyridyl)amino)ethoxy]benzyl]thiazolidine-2,4-dione, potassium salt

A solution of potassium hydroxide (4.71 g) in water (5.0 ml) was added to a stirred suspension of 5-[4-[2-(*N*-methyl-*N*-(2-pyridyl)amino)ethoxy]benzyl]thiazolidine-2,4-dione (25.0 g) in propan-2-ol (250 ml) at reflux. The stirred mixture was maintained at reflux for 15 minutes and then cooled to 21 °C over approximately 1 hour. The solid was collected by filtration, washed with propan-2-ol (50 ml) and dried under vacuum at 60°C for 16 hours to afford the 5-[4-[2-(*N*-methyl-*N*-(2-pyridyl)amino)ethoxy]benzyl] thiazolidine-2,4-dione, potassium salt (26.6 g) as a white crystalline solid.

### Characterising data recorded for the product of Example 3

The infrared absorption spectrum of a mineral oil dispersion of the product was obtained using a Nicolet 710 FT-IR spectrometer at 2 cm⁻¹ resolution (Figure 1). Data were digitised at 1 cm⁻¹ intervals. Bands were observed at: 1668, 1605, 1596, 1559, 1537, 1512, 1504, 1424, 1311, 1263, 1247, 1224, 1206, 1199, 1178, 1156, 1061, 1008, 977, 964, 896, 830, 783, 764, 746, 731, 692, 663, 559, 510, 479 cm⁻¹.

The infrared spectrum of the solid product was recorded using a Perkin-Elmer Spectrum One FT-IR spectrometer fitted with a universal ATR accessory. Bands were observed at: 2924, 2867, 1667, 1595, 1557, 1534, 1501, 1462, 1438, 1422, 1389, 1364, 1309, 1262, 1244, 1220, 1206, 1197, 1178, 1155, 1106, 1080, 1060, 1007, 977, 963, 922, 896, 829, 782, 764, 746, 729, 692, 662 cm⁻¹.

The Raman spectrum (Figure 2) was recorded with the sample in an NMR tube using a Nicolet 960 E.S.P. FT-Raman spectrometer, at 4 cm⁻¹ resolution with excitation from a Nd:V04 laser (1064 nm) with a power output of 400 mW. Bands were observed at: 3068, 3055, 3012, 2925, 2900, 2868, 1663, 1611, 1560, 1463, 1439, 1424, 1387, 1313, 1275, 1206, 1179, 1158, 1099, 1057, 977, 923, 897, 842, 783, 750, 726, 663, 633, 480, 405, 347 cm⁻¹.

The X-Ray Powder Diffractogram pattern of the product (Figure 3) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 1.

**Table 1**

| **Angle** | **Rel. Intensity** |
|---|---|
| **2-Theta°** | **%** |
| 3.1 | 100 |
| 6.2 | 13 |
| 9.3 | 13.2 |
| 12.1 | 0.8 |
| 12.5 | 3.1 |
| 12.9 | 1 |
| 13.5 | 1.3 |
| 14.3 | 8 |
| 15.2 | 35.9 |
| 15.6 | 2.9 |
| 16.3 | 1.4 |
| 17.4 | 0.9 |
| 18.5 | 3.6 |
| 18.8 | 10.5 |
| 19.8 | 2.9 |
| 20.1 | 2.9 |
| 20.5 | 2.4 |
| 21.1 | 6.2 |
| 21.7 | 1.3 |
| 22.4 | 9.3 |
| 23.3 | 11.3 |
| 23.8 | 7.3 |
| 24.2 | 8.4 |
| 24.9 | 2.9 |
| 25.5 | 3.2 |
| 26.6 | 4.4 |
| 27.1 | 9.9 |
| 28.0 | 5.8 |
| 28.4 | 4.5 |
| 29.6 | 4.3 |
| 29.9 | 2.6 |
| 30.9 | 6.1 |
| 32.1 | 10.1 |
| 32.8 | 2.5 |
| 33.2 | 7.3 |
| 34.5 | 3.1 |

The solid-state NMR spectrum of the product (Figure 4) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 38.8, 49.7, 64.3, 66.7, 103.9, 110.9, 118.1, 129.1, 131.1, 132.4, 136.2, 148.3, 158.6, 191.1, 196.3 ppm.

### Properties of the Potassium Salt, recorded for the product of Example 5

### Solid State Stability of the Potassium Salt

The solid state stability of the drug substance was determined by storing approximately 1.0 g of the material in a glass bottle at a) 40°C / 75 % Relative Humidity (RH), open exposure, for 1 month and b) at 50°C, closed, for 1 month. The material was assayed by HPLC for final content and degradation products in both cases.
a) 40°C / 75% RH: No significant degradation observed (HPLC assay 101 % initial).
b) 50°C: No significant degradation observed (HPLC assay 99% initial).

### Solubility of the Potassium Salt

The solubility of the material was determined by adding water in aliquots from 1 to 1000ml to approximately 100mg of drug substance until the powder had dissolved. The visual solubility was confirmed by an HPLC assay of a saturated solution. Solubility: > 100 mg/ml.

### Melting Range of the Potassium Salt

The melting range of the Potassium Salt was determined according to the method described in the U.S. Pharmacopoeia, USP 23, 1995, <741> "Melting range or temperature, Procedure for Class Ia", using a Buchi 545 melting point instrument.
Melting Range: 196.4-200.6°C

### Tₒₙₛₑₜ of the Potassium Salt

The Tₒₙₛₑₜ of the drug substance was determined by Differential Scanning Calorimetry using a Perkin-Elmer DSC7 apparatus.
Tₒₙₛₑₜ (10°C/minute, closed pan): 205°C

## Claims

1. A compound 5-[4-[2-(N-methyl-N-(2-pyridyl)amino)ethoxy]benzyl]thiazolidine-2,4-dione potassium salt or a pharmaceutically acceptable solvate thereof, **characterised in that** it provides one or more of:
(i) an infrared spectrum substantially in accordance with Figure 1;
(ii) a Raman spectrum substantially in accordance with Figure 2;
(iii) an X-Ray powder diffraction pattern (XRPD) substantially in accordance with Table 1 or Figure 3; and
(iv) a Solid State ¹³C NMR spectrum substantially in accordance with Figure 4; and
(v) a melting point in the range of 194 to 201°C.

2. A compound according to claim 1, **characterised in that** it provides two or more of:
(i) an infrared spectrum substantially in accordance with Figure 1;
(ii) a Raman spectrum substantially in accordance with Figure 2;
(iii) an X-Ray powder diffraction pattern (XRPD) substantially in accordance with Table 1 or Figure 3; and
(iv) a Solid State ¹³C NMR spectrum substantially in accordance with Figure 4.

3. A compound according to claim 1, in a solid dosage form.

4. A compound according to claim 1 or claim 2, in a bulk milled form.

5. A pharmaceutical composition comprising 5-[4-[2-(N-methyl-N-(2-pyridyl)amino)ethoxy]benzyl]thiazolidine-2,4-dione potassium salt (the Potassium Salt) or a pharmaceutically acceptable solvate thereof, according to claim1, wherein the Potassium Salt is present in an amount providing 1, 2, 3. 4, 4 to 8 or 8 to 12mg of 5-[4-[2-(N-methyl-N-(2-pyridyl)amino)ethoxy]benzyl]thiazolidine-2,4-dione and a pharmaceutically acceptable carrier therefor.

6. A pharmaceutical composition according to claim 5, comprising the Potassium Salt or a pharmaceutically acceptable solvate thereof in an amount providing 1, 2, 3 or 4mg of 5-[4-[2-(N-methyl-N-(2-pyridyl)amino)ethoxy]benzyl]thiazolidine-2,4-dione.

7. A pharmaceutical composition according to claim 5, comprising the Potassium Salt or a pharmaceutically acceptable solvate thereof in an amount providing 2mg of 5-[4-[2-(N-methyl-N-(2-pyridyl)amino)ethoxy]benzyl]thiazolidine-2,4-dione.

8. A pharmaceutical composition according to claim 5, comprising the Potassium Salt or a pharmaceutically acceptable solvate thereof in an amount providing 4mg of 5-[4-[2-(N-methyl-N-(2-pyridyl)amino)ethoxy]benzyl]thiazolidine-2,4-dione.

9. A pharmaceutical composition according to claim 5, in an amount providing 8mg of 5-[4-[2-(N-methyl-N-(2-pyridyl)amino)ethoxy]benzyl.]thiazolidine-2,4-dione.

10. A pharmaceutical composition comprising 5-[4-[2-(N-methyl-N-(2-pyridyl)amino)ethoxy]benzyl]thiazolidine-2,4-dione potassium salt or a pharmaceutically acceptable solvate thereof in combination with one or more other anti-diabetic agents and optionally a pharmaceutically acceptable carrier therefor.

11. A method for the treatment and/or prophylaxis of diabetes mellitus, conditions associated with diabetes mellitus and certain complications thereof, in a human or non-human mammal which comprises administering an effective, non-toxic, amount of 5-[4-[2-(N-methyl-N-(2-pyridyl)amino)ethoxy]benzyl]thiazolidine-2.4-dione potassium salt or a pharmaceutically acceptable solvate thereof to a human or non-human mammal in need thereof, wherein the Potassium Salt is present in an amount providing 1, 2, 3, 4, 4 to 8 or 8 to 12mg of 5-[4-[2-(N-methyl-N-(2-pyridyl)amino)ethoxy]benzyl]thiazolidine-2,4-dione.

12. A method for the treatment and/or prophylaxis of diabetes mellitus, conditions associated with diabetes mellitus and certain complications thereof, in a human or non-human mammal which comprises administering an effective, non-toxic, amount of 5-[4-[2-(N-methyl-N-(2-pyridyl)amino)ethoxy]benzyl]thiazolidine-2,4-dione potassium salt or a pharmaceutically acceptable solvate thereof in combination with one or more other anti-diabetic agents.

13. A compound 5-[4-[2-(N-methyl-N-(2-pyridyl)amino)ethoxy]benzyl]-thiazolidine-2,4-dione potassium salt or a pharmaceutically acceptable solvate thereof for use as an active therapeutic substance wherein the Potassium Salt is present in an amount providing 1, 2, 3, 4, 4 to 8 or 8 to 12mg of 5-[4-[2-(N-methyl-N-(2-pyridyl)-amino)ethoxy]benzyl]thiazolidine-2,4-dione.

14. A use of 5-[4-[2-(N-methyl-N-(2-pyridyl)amino)ethoxy]benzyl]thiazolidine-2,4-dione potassium salt or a solvate thereof, for the manufacture of a medicament for the treatment and/or prophylaxis of diabetes mellitus, conditions associated with diabetes mellitus and certain complications thereof, wherein the Potassium Salt is present in an amount providing 1, 2, 3. 4, 4 to 8 or 8 to 12mg of 5-[4-[2-(N-methyl-N-(2-pyridyl)amino)ethoxy]benzyl]thiazolidine-2,4-dione.
